# EUROPEAN PATENT APPLICATION

(11) **EP 1 477 161 A1**
(43) Date of publication of application: **17.11.2004**
(21) Application number: 04011541.2
(22) Date of filing: 14.05.2004
(51) Int. Cl.: A61K 9/16, A61K 9/20, A61K 9/28

(54) **Pharmaceutical solid preparation containing a poorly soluble drug and production process thereof**

(30) Priority: 16.05.2003 JP 2003138566
(71) Applicant: Shin -Etsu Chemical Co. Ltd., Tokyo (JP)
(72) Inventor: Tanno, Fumie, Specialty Chem. Res. Center, Kubiki-mura Naka Kubiki-gun Niigata-ken (JP); Nishiyama, Yuichi, Specialty Chem. Res. Center, Kubiki-mura Naka Kubiki-gun Niigata-ken (JP)
(74) Representative: Reinhard - Skuhra - Weise & Partner

(57) **Abstract**

Among the conventional methods for producing a solid dispersion, the solvent method is renowned for providing a solid dispersion that has improved solubility and bioavailability for poorly soluble drugs. However, the solvent method often uses volatile organic solvents such as dichloromethane, acetone and alcohol, causing problems such as the organic solvents remaining in the product, environmental pollution, workplace safety as well as the cost of the equipment necessary to avoid these concerns. Accordingly, a novel process is provided for preparing a solid dispersion without using the organic solvents often used in a conventional solvent method. That is, it is found that a solid preparation excellent in solubility can be obtained by carrying out a non-solvent coating method in which a core particle or particles are coated with an enteric coating agent and a plasticizer composition comprising a poorly soluble drug dissolved in a plasticizer.

## Description

### BACKGROUND OF THE INVENTION

### 1. Field of the Invention

The present invention relates to a process for producing a solid preparation containing a poorly soluble drug for the purpose of improvement of dissolution. More particularly, the present invention relates to a preparation which is coated with a non-solvent enteric coating agent and a plasticizer composition comprising a poorly soluble drug dissolved in a plasticizer.

### 2. Description of the related art

In recent years a number of newly developed drugs have had poor solubility, which has made improvements in their solubility or bioavailability an important matter. To increase a drug's solubility or bioavailability various production processes such as drug micronization or amorphization, or making the drug into a solid dispersion have been proposed. Of these, the process of making the drug into a solid dispersion is of dispersing the drug into an inert carrier, and as such has been widely investigated.

While many processes have been proposed for a solid dispersion production process, the solvent method can be cited as a particularly practical process.

There are two types of solvent method. One is where a drug and a water-soluble polymer that is acting as a carrier are dissolved into a solvent such as an organic solvent, after which the solvent is evaporated off to obtain the solid dispersion. The other is where the drug is dissolved in a solvent and dispersed into a carrier, after which the solvent is evaporated off to obtain the solid dispersion. By dissolving a poorly soluble drug into a solvent the drug becomes amorphous and disperses into the carrier in this amorphous state, whereby it is thought solubility and bioavailability improve.

Specific examples of the solvent method include those of Japanese Patent Application Examined Publication Nos. 3-001288/1991 and 3-028404/1991, which disclose that lactose or the like was granulated with a water-soluble polymer such as hydroxypropylcellulose to make fine.particles. The poorly soluble drug nifedipine and a polymer base such as poly(vinylpyrrolidone), hydroxypropylmethylcellulose and methylcellulose were dissolved in an organic solvent to form a solution, which was sprayed on the fine particles. The spray-coated particles were dried to yield the solid dispersion.

In addition, Japanese Patent Application Unexamined Publication No. 2000-281561 discloses a solid dispersion which was prepared by dissolving a poorly soluble drug such as cycloheptadine and a water-soluble polymer such as poly(vinylpyrrolidone), hydroxypropylmethylcellulose and hydroxypropylcellulose in a water/alcohol system, and subsequently spraying on lactose for granulating.

Japanese Patent Application Unexamined Publication No. 11-116502/1999 discloses a solid dispersion obtained by a process wherein a composition containing a poorly soluble drug and hydroxypropylcellulose acetate succinate was dissolved in an organic solvent, wherein rather than spraying onto a carrier such as lactose, the solution was then spray-dried in an air-flow of a direct fluid bed.

### SUMMARY OF THE INVENTION

Among conventional processes for producing a solid dispersion, dispersions obtained by the solvent method have good improvements in terms of solubility and bioavailability of the poorly soluble drug. However, because volatile organic solvents such as dichloromethane, acetone and alcohol are often used in the solvent method, problems arise with organic solvent residue in products, environmental pollution and workplace safety, as well as industrial problems relating to the capital investment required to avoid such concerns.

As a result of intensive study to solve the above problems, the present inventors have found a method for preparing a solid dispersion without use of an organic solvent, which are frequently used in the conventional solvent method. That is, the inventors have arrived at the present invention by finding that a solid preparation having excellent solubility can be obtained through non-solvent coating by dissolving a poorly soluble drug in a plasticizer to form a plasticizer composition, and then coating a core particle or particles with the plasticizer composition and a fine powder enteric coating agent. According to the present invention, a solid preparation can be prepared by using existing equipment and techniques without using a conventional organic solvent. It was also confirmed that the resultant solid preparation affords a similar improvement effect of dissolution as those of the conventional solvent methods.

Specifically, the present invention provides a production process for an enteric solid pharmaceutical preparation which a core particle or particles are coated with a powder enteric coating agent, while spraying a plasticizer composition comprising a poorly soluble drug dissolved in a plasticizer. The present invention also provides an enteric solid pharmaceutical preparation comprising a core particle or particles, a plasticizer composition in which a poorly soluble drug is dissolved in a plasticizer, and a powder enteric coating agent, wherein the core particle or particles are coated with the plasticizer composition and the powder enteric coating agent.

According to the present invention, an enteric solid preparation which has improved dissolution of a poorly soluble drug can be produced in a simple manner with existing equipment without using an organic solvent. Furthermore, the solid preparation according to the present invention has the same dissolution properties as that of a solid preparation obtained by a conventional solvent method.

### DETAILED DESCRIPTION OF THE PREFERRED EMBODIMENTS

The poorly soluble drug used in the present invention may be a drug which has extremely low solubility in water and which has inferior bioavailability in normal oral administration, and may be referred to, for example, as the drug defined as "nearly insoluble" or "extremely difficult to be dissolved" in the Japanese Pharmacopoeia. In the Japanese Pharmacopoeia, when a drug is in solid form the solubility of the drug is defined as the degree of dissolvability within 30 minutes when the drug is placed in a solvent after being made into a powder, and shaken for 30 seconds every 5 minutes at 20 ± 5°C. "Nearly insoluble" is referred to the characteristic where an amount of 10,000 ml or more of the solvent is required to dissolve 1 g or 1 ml of the drug. "Extremely difficult to be dissolved" is referred to the characteristic where a solvent amount of 1,000 to 10,000 ml is required to dissolve 1 g or 1 ml of the drug. Specifically, for example, such drugs may include ibuprofen, indomethacin, nifedipine, phenacetin, phenytoin, digitoxin, digoxin, nilvadipine, diazepam, griseofulvin, chloramphenicol and sulfathiazole.

The plasticizer used in the present invention may not be particularly limited as long as it is able to dissolve the poorly soluble drug at room temperature or by heating at 90°C or less, is hydrophobic and can fuse to the fine powder enteric coating agent. Examples may include propylene glycol, triethyl citrate, polyethylene glycol, acetyl monoglyceride, glycerine, tributyl citrate, triacetin, diacetin, monoacetin and diethyl phthalate, among which triethyl citrate and polyethylene glycol may be preferable. The above plasticizers may be used alone or in combination of two or more. These plasticizers may be added to improve the plasticity of the coating film and the homogeneity of the film formation. The amount added may not be particularly limited as long as it is an amount sufficient to achieve these purposes. However, it may be desirable that the minimum weight part to sufficiently dissolve the drug in the plasticizer is added ranging from 1 to 20 weight parts, and preferably from 7 to 15 weight parts per one weight part of the soluble drug used.

Adding a surfactant, oil, higher alcohol, higher fatty acid, a hydrophobic wax such as glycerin fatty acid ester or the like may be also effective for improving the solubility of the drug in the plasticizer.

A core particle or particles (carrier) to be preferably usable in the present invention, which will be coated with the fine powder enteric coating agent being dispersed, may include, for example, powders or cores of lactose, sucrose, glucose, trehalose, fructose, dextrin, starch, pullulan, carboxymethylcellulose and salts thereof, carboxymethylstarch and salts thereof, cellulose, crystalline cellulose, polyvinyl alcohol and hemicellulose. Further examples may include spherical granules of white sugar-starch, spherical granules of refined white sugar and spherical granules of lactose-crystalline cellulose as well as granules or fine particles of solid preparations.

The average particle diameter of the core particles may be preferably 75 to 1700 µm, and more preferably 100 to 1400 µm. It may be preferable if the core particles are undercoated with one or more appropriately selected from hydroxypropylmethylcellulose, hydroxypropylcellulose, ethylcellulose, shellac and the like. From a perspective of protection of the core particles under the coat and homogeneity of the coating film, the undercoating amount may be preferably 1 to 10% by weight of the weight of the core particles.

The enteric coating used in the present invention may be a fine powder preferably having an average particle diameter of 10 µm or less, and more preferably 1 to 10 µm. Examples of cellulose derivatives which can be used may include cellulose acetate phthalate (CAP), cellulose acetate trimellitate (CAT), hydroxypropylmethylcellulose phthalate (HPMCP), hydroxypropylmethylcellulose acetate succinate (HPMCAS), hydroxypropylmethylcellulose trimellitate (HPMCT), hydroxypropylmethylcellulose acetate maleate (HPMCAM) and carboxymethylethylcellulose (CMEC). Acrylics such as copolymer of methacrylic acid and ethyl acrylate can also be used. The enteric coating agent used in the present invention may preferably comprise a cellulose derivative, and may further preferably comprise one or more selected from the group consisting of hydroxypropylmethylcellulose acetate succinate (HPMCAS), hydroxypropylmethylcellulose trimellitate (HPMCT), and hydroxypropylmethylcellulose acetate maleate (HPMCAM). If the average particle diameter exceeds 10 µm, the coating agent may not properly fuse to the granules. This may mean that a solid preparation in which the drug is homogenously dispersed cannot be obtained.

Among these coating agents, hydroxypropylmethylcellulose acetate succinate (HPMCAS), which has a low softening temperature and excellent film-forming properties, may be preferable.

Further, by combining with a fine powder of a polymer other than the enteric coating agent, for example ethylcellulose or an acrylic polymer, the preparation can be made into a strained release preparation.

According to the present invention, a large-scale drying capacity may not be required because a solvent is not used. For extension and fusion of the dispersed fine powder enteric coating agent as well as satisfactory dispersion of the drug, a certain amount of heating and stirring capacity may be preferable. For example, such capacity may be provided by a centrifugal fluid apparatus, pan coating apparatus and fluidized bed coating apparatus. Among these coating apparatuses, a centrifugal fluid apparatus may be more suitable.

According to the production process for a solid preparation of the present invention, for example, a poorly soluble drug is dissolved in a liquid plasticizer at room temperature or in a melted liquid plasticizer. The obtained solution is sprayed (for example, using a spray or the like) to core particles of lactose which are being stirred and rolled in an above-mentioned centrifugal rolling apparatus, while simultaneously an enteric coating agent having an average particle diameter of 10 µm or less is dispersedly added to said core particles. This series of operations can also be performed by splitting into several steps while varying the compositions. The talc, carplex (SiO₂), magnesium stearate, cornstarch or the like may be also dispersedly added simultaneously to prevent the granules from sticking to each other during coating.

In the present invention the weight ratio between the plasticizer and the enteric coating agent, and the coating amount for the core particles, are important factors in improving solubility.

The weight ratio between the plasticizer and the enteric coating agent may be preferably in the range of approximately plasticizer : enteric coating agent = (2 : 8) to (8 : 2).

A preferable coating amount of the enteric coating agent may be preferably in the range of approximately 10 to 50% by weight based on the weight of core particles.

The solid preparation obtained according to the above may also be subsequently coated again with another polymer compound. A pharmaceutically allowable drug or additive such as plasticizer, colorant, pigment, anti-sticking agent (talc) or oil may also be added for the coating.

Moreover, as the granule anti-adhering agent subsequent to coating, one or more selected from the group consisting of an inorganic substance or metal salt of an organic acid such as talc, carplex, magnesium stearate and calcium stearate; a water-soluble polymer such as hydroxypropylmethylcellulose, hydroxypropylcellulose and polyethylene glycol; and a wax such as carnauba wax, white beeswax and paraffin can also be used for further coating the obtained solid preparation.

Hereinafter, the present invention will be further explained in detail using examples. However, the present invention is not to be restricted to these examples alone.

### Example 1

Prior to producing the solid preparation according to the present invention, 500 g of core granules (Nonpareil 101, 20^{#} to 24^{#}, manufactured by Freund Industrial Co., Ltd.) was placed in a centrifugal flow coating apparatus (CF Coater CF 360, manufactured by Freund Industrial Co., Ltd.). And, an aqueous 5% by weight solution of hydroxypropylmethylcellulose (TC-5R, manufactured by Shin-Etsu Chemical Co., Ltd.) was sprayed thereto so that the hydroxypropylmethylcellulose amount on the core granules was 5% by weight. Thus, the undercoating was accomplished.

The core granules prepared in this way were placed in a centrifugal rolling apparatus (CF Coater CF360, manufactured by Freund Industrial Co., Ltd.) in an amount of 500 g.

The 25 g poorly soluble nifedipine was dissolved in the plasticizers of 75 g triethyl citrate and 175 g polyethylene glycol 400 to produce a mixed solution. The solution was sprayed to the core granules at a rate of 13.75 g/min, while the rolling apparatus was held at an intake air temperature of 60°C, product temperature of 40°C and revolution of 200 rpm. And simultaneously, the uniformly mixed powders containing 250 g of hydroxypropylmethylcellulose acetate succinate and 150 g of talc were added to the core granules at a rate of 20 g/min. The hydroxypropylmethylcellulose acetate succinate was supplied by Shin-Etsu Chemical Co., Ltd. (product AS-MF), had average particle diameter of 5 µm and contained succinoyl group 11.0% by weight, acetyl group 9.3% by weight, hydroxypropoxyl group 7.4% by weight and methoxyl group 23.0% by weight.

Subsequently, 20 g of carplex (light anhydrous silicic acid) was sprayed as an anti-adhering agent for the granules and stirred as the so-called post-dry for 10 minutes at product temperature of 40°C. The yield was 95% and the treatment time 30 minutes.

A dissolution test was performed on the obtained solid preparation using "the Second Fluid of the disintegration test" in Japanese Pharmacopoeia 12. In the test, when a sample of nifedipine in an amount corresponding to 50 µg/mL was added, the nifedipine dissolution rate after 60 minutes was 45 µg/mL. This figure is 10 µg/mL higher than the solubility of nifedipine by itself, showing that the drug in solution was in a super-saturated state. Thus, the dissolution of the drug for the solid preparation prepared according to the present invention was improved.

### Example 2

A solid preparation was prepared in the same manner, except that the hydroxypropylmethylcellulose acetate succinate used in Example 1 was replaced with hydroxypropylmethylcellulose acetate succinate. The hydroxypropylmethylcellulose acetate succinate was supplied by Shin-Etsu Chemical Co., Ltd. (product AS-LF), had average particle diameter of 5 µm and contained succinoyl group: 14.8% by weight, acetyl group: 7.3% by weight, hydroxypropoxyl group: 7.1% by weight and methoxyl group: 22.7% by weight.

A dissolution test using the Second Fluid Of JP12 was performed on the obtained solid preparation in the same manner as in Example 1. Consequently, the nifedipine dissolution rate after 60 minutes was 45 µg/mL. This figure is 10 µg/mL higher than the solubility of nifedipine by itself, showing that the drug in solution was in a super-saturated state. Thus, the dissolution of the drug for the solid preparation prepared according to the present invention was improved.

### Example 3

A solid preparation was prepared in the same manner, except that the hydroxypropylmethylcellulose acetate succinate used in Example 1 was replaced with hydroxypropylmethylcellulose acetate succinate. The hydroxypropylmethylcellulose acetate succinate was supplied by Shin-Etsu Chemical Co., Ltd. (product AS-HF), had average particle diameter of 5 µm and contained succinoyl group: 7.8% by weight, acetyl group: 11.1% by weight, hydroxypropoxyl group: 7.4% by weight and methoxyl group: 23.5% by weight.

A dissolution test using the Second Fluid of JP12 was performed on the obtained solid preparation in the same manner as in Example 1. Consequently, the nifedipine dissolution rate after 60 minutes was 45 µg/mL. This figure is 10 µg/mL higher than the solubility of nifedipine by itself, showing that the drug in solution was in a super-saturated state. Thus, the dissolution of the drug for the solid preparation prepared according to the present invention was improved.

### Example 4

The 500g of core granules prepared in Example 1 were placed in a centrifugal rolling apparatus (CF Coater CF360, manufactured by Freund Industrial Co., Ltd.).

The 25 g of poorly soluble nifedipine was dissolved at 60°C in the mixed plasticizer liquids of 17.5 g of triethyl citrate, 10 g of Myvacet (glycerin fatty acid ester) and 230 g of polyethylene glycol 6000. The solution was sprayed at a rate of 28.25 g/min, while the rolling apparatus was held at an intake air temperature of 60°C, product temperature of 40°C and revolution of 200 rpm with. And simultaneously, uniformly mixed powders containing 50 g of hydroxypropylmethylcellulose acetate succinate and 30 g of talc were added at a rate of 8 g/min.

Subsequently, 20 g of carplex (light anhydrous silicic acid) was dispersedly added as an anti-adhering agent for the granules and stirred for 10 minutes at product temperature of 40°C. The yield was 87% and the treatment time 20 minutes.

A dissolution test was performed on the obtained solid preparation using the Second Fluid of the disintegration test in Japanese Pharmacopoeia 12. In the test, when a sample of nifedipine in an amount corresponding to 50 µg/mL was added, the nifedipine dissolution rate after 60 minutes was 40 µg/mL. This figure is 10 µg/mL higher than the solubility of nifedipine by itself, showing that the drug in solution was in a super-saturated state. Thus, the dissolution of the drug for the solid preparation prepared according to the present invention was improved.

### Example 5

A solid preparation was prepared in the same manner except that the hydroxypropylmethylcellulose acetate succinate used in Example 1 was replaced with hydroxypropylmethylcellulose trimellitate (average particle diameter 5 µm, Shin-Etsu Chemical Co., Ltd. product HPMCT).

A dissolution test using the Second Fluid of JP12 was performed on the obtained solid preparation in the same manner as in Example 1. Consequently, the nifedipine dissolution rate after 60 minutes was 30 µg/mL, showing that the drug in solution was in a super-saturated state. Thus, the dissolution of the drug for the solid preparation prepared according to the present invention was improved.

### Comparative Example 1

The 500g of core granules prepared in Example 1 were placed in a centrifugal rolling apparatus (CF Coater CF360, manufactured by Freund Industrial Co., Ltd.).

The powders of 25 g of the poorly soluble drug nifedipine and 50 g of hydroxypropylmethylcellulose acetate succinate (AS-MF) were dissolved in 700g of a mixed solvent of dichloromethane and ethanol (weight ratio 1:1). The solution was sprayed at a rate of 20 g/min, while the rolling apparatus was held at an intake air temperature of 40°C, product temperature of 36°C and revolution of 200 rpm. Thus, a solid preparation was prepared in accordance with the solvent method using a conventional organic solvent.

A dissolution test using the Second Fluid of JP12 was performed on the obtained solid preparation in the same manner as in Example 1. Consequently, the nifedipine dissolution rate after 60 minutes was 45 µg/mL.

### Comparative Example 2

A solid preparation was prepared in the same manner as that in Comparative Example 1, except that hydroxypropylmethylcellulose trimellitate was used in place of the hydroxypropylmethylcellulose acetate succinate of Comparative Example 1.

A dissolution test using the Second Fluid of JP-12 was performed on the obtained solid preparation in the same manner as in Example 1. Consequently, the nifedipine dissolution rate after 60 minutes was 30 µg/mL.

It was learned from the above results that a solid preparation prepared in accordance with the production process of the present invention improves dissolution of a poorly soluble drug in the same way as that of a solid preparation obtained by a conventional method.

## Claims

1. An enteric solid preparation comprising a core particle or particles, a plasticizer composition comprising a poorly soluble drug dissolved in a plasticizer, and a powder enteric coating agent, wherein the core particle or particles are coated with the plasticizer composition and the powder enteric coating agent.

2. The enteric solid preparation according to claim 1, wherein said plasticizer is ethyl citrate or polyethylene glycol.

3. The enteric solid preparation according to claim 1 or 2, wherein said enteric coating agent has an average particle diameter of 10 µm or less.

4. The enteric solid preparation according to any of claims 1 to 3, wherein said enteric coating agent comprises a cellulose derivative.

5. The enteric solid preparation according to any of claims 1 to 4, wherein said enteric coating agent comprises a compound selected from the group consisting of hydroxypropylmethylcellulose acetate succinate, hydroxypropylmethylcellulose trimellitate, and hydroxypropylmethylcellulose acetate maleate.

6. A production process for an enteric solid preparation comprising a step of coating a core particle or particles with a powder enteric coating agent, while spraying a plasticizer composition comprising a poorly soluble drug dissolved in a plasticizer.
